(19) **Europäisches Patentamt**

**European Patent Office**

**Office européen des brevets**

(11) **EP 1 074 261 A1**

(12) **EUROPEAN PATENT APPLICATION**
published in accordance with Art. 158(3) EPC

(43) Date of publication:
07.02.2001 Bulletin 2001/06

(21) Application number: 99918291.8

(22) Date of filing: 30.04.1999

(51) Int. Cl.$^7$: **A61K 33/18**, A61K 47/30

(86) International application number:
PCT/JP99/02328

(87) International publication number:
WO 99/56757 (11.11.1999 Gazette 1999/45)

(84) Designated Contracting States:
**DE FR GB IT NL**

(30) Priority: 30.04.1998 JP 12023298

(71) Applicants:
- **FUJISAWA PHARMACEUTICAL CO., LTD.**
  **Osaka-shi Osaka 541-8514 (JP)**
- **SANYO CHEMICAL INDUSTRIES, LTD.**
  **Kyoto-shi, Kyoto 605-0995 (JP)**

(72) Inventors:
- **KOMATSU, Takayoshi**
  **Tsukuba-shi, Ibaraki 305-0042 (JP)**
- **SHINODA, Katsumi**
  **Sanyo Chemical Industries, Ltd.**
  **Kyoto-shi Kyoto 605-0995 (JP)**
- **KATAOKA, Yoshimi**
  **Sanyo Chemical Industries, Ltd.**
  **Kyoto-shi, Kyoto 605-0995 (JP)**

(74) Representative:
**Gille Hrabal Struck Neidlein Prop Roos**
**Patentanwälte,**
**Brucknerstrasse 20**
**40593 Düsseldorf (DE)**

(54) **IODOPHOR COMPOSITIONS FOR PREVENTING MASTITIS**

(57)     The present invention has its object to provide an iodophor composition for the prevention of mastitis which is hard to freeze in winter and has an improved antibacterial effect, equal to the conventional dilution, even after dilution at a high dilution factor.

The present invention is directed to an iodophor composition for the prevention of mastitis comprising iodine, an iodide, an organic acid, a nonionic surfactant having the HLB number of 6 to 15 and water,
wherein the viscosity (VI) of a 10-fold dilution of the composition is 20 to 3000 mm$^2$/s and

the ratio of the viscosity (V2) of the composition as such to the viscosity (V1) of said dilution is (V2)/(V1) = 0.05 to 20.

**EP 1 074 261 A1**

# EP 1 074 261 A1

## Description

TECHNICAL FIELD

The present invention relates to an iodophor composition for topical antiasepsis of farm animals, particularly for the prevention of mastitis in farm animals.

BACKGROUND ART

Heretofore, as an iodophor composition for the prevention of mastitis in cows, and the like, an aqueous solution (hereinafter referred to as stock solution) of a complex comprising iodine, an iodide, an iodine-complexing polymer, a surfactant, an alcohol, a polyol, etc. with a water content of about 70 to 90 weight % has been employed and diluted 1~4-fold with tap water in the field.

The common practice for preventing mastitis comprises dipping the teats and udder in a dilution of the stock solution for a few seconds immediately after milking to destroy the bacteria which fur the teats and udder. In addition, it is necessary to prevent the invasion of air-borne bacteria from the teat orifices during the intervening period following dipping to the time of closure of the teat orifices. Therefore, in order to insure sustained sterility after dipping, the stock solution is formulated so that the viscosity of the solution as diluted will usually be 20 to 70mm$^2$/s and, hence, an effective amount of iodine may be deposited on the teats.

However, the preparation is often used in cold climates and tends to get frozen in the winter months causing difficulties in handling and, moreover, its freezing results in phase separation. Furthermore, with the increasing age of dairy farmers who are consumers, they are experiencing inconveniences in the transport of heavy cans or drums, particularly in loading and unloading the vehicles with them. Furthermore, because of the low dilution factor recommended, the transportation of the stock solution is substantially equivalent to the transportation of a large quantity of water and the cost of transportation is inevitably enormous.

SUMMARY OF THE INVENTION

The inventors of the present invention did intensive research and the present invention has been accomplished.

The present invention, therefore, is directed to an iodophor composition for the prevention of mastitis comprising iodine (A), an iodide (B), an organic acid (C), an iodine carrier (D) and water,

wherein the viscosity (V1) of a 10-fold dilution of the composition is 20 to 3000 mm$^2$/s and

the ratio of the viscosity (V2) of the composition as such to the viscosity (V1) of said dilution is (V2)/(V1) = 0.05 to 20.

DISCLOSURE OF INVENTION

In the present invention, the iodide (B) is not particularly restricted as far as it dissociates an iodide ion in water, and includes metal iodides such as alkali metal iodides (e.g. sodium iodide, potassium iodide) and alkaline earth metal iodides (e.g. calcium iodide, magnesium iodide). Among these, preferred are alkali metal iodides.

The organic acid (C) is not particularly restricted, either, as far as it is a water-soluble organic acid (solubility: 0.1 weight % or more at 25 °C), but includes carboxylic acids, sulfonic acids, sulfinic acids and anionic surfactants.

The carboxylic acids mentioned above include monocarboxylic, polycarboxylic and hydroxycarboxylic acids.

The monocarboxylic acids include aliphatic saturated or unsaturated carboxylic acids containing 1 to 8 carbon atoms and aromatic saturated or unsaturated carboxylic acids (e.g. formic acid, acetic acid, propionic acid, benzoic acid). The polycarboxylic acids include dihydric to polyhydric carboxylic acids, and the dihydric carboxylic acids include aliphatic saturated or unsaturated carboxylic acids containing 2 to 10 carbon atoms and aromatic saturated or unsaturated carboxylic acids (e.g. oxalic acid, malonic acid, glutaric acid, adipic acid, maleic acid), and the polycarboxylic acids more functional than dihydric include trimellitic acid. The hydroxycarboxylic acids include aliphatic saturated or unsaturated hydroxycarboxylic acids containing 2 to 10 carbon atoms and aromatic saturated or unsaturated hydroxycarboxylic acids (e.g. glycolic acid, lactic acid, malic acid, citric acid, salicylic acid). Further, other carboxylic acids include aminocarboxylic acids such as glycine, sarcosine, arginine, etc. Among them, preferred are dihydric carboxylic acids and hydroxycarboxylic acids. More preferred are glutaric acid, adipic acid, lactic acid, malic acid and citric acid. Particularly preferred is citric acid.

The sulfonic acids include aliphatic sulfonic acids and aromatic sulfonic acids. The aliphatic sulfonic acids include methanesulfonic acid and ethanesulfonic acid, among others. Said aromatic sulfonic acids include toluenesulfonic acid and naphthalenesulfonic acid, among others. The sulfinic acids include aliphatic sulfinic acids (e.g. butanesulfinic acid) and aromatic sulfinic acids (e.g. benzenesulfinic acid).

The anionic surfactants include sulfate esters, carboxymethyl compounds and phosphate esters.

Said sulfate esters include higher alcohol sulfate esters (sulfate esters of aliphatic alcohols containing 8 to 18 carbon atoms), higher alkyl ether sulfate esters [sulfate esters of $C_{8-18}$ aliphatic alcohol-ethylene oxide (hereinafter referred as to EO) (1 to 10 moles) adducts], sulfated oils (naturally-occurring unsaturated oils, fats or waxes directly sulfated), sulfated fatty acid esters (sulfated unsaturated fatty acid lower alcohol esters) and sulfated olefins (sulfated $C_{8-18}$ olefins).

A specific example of said higher alcohol sulfate esters includes octyl alcohol sulfate ester, decyl alcohol sulfate ester, lauryl alcohol sulfate ester, stearyl alcohol sulfate ester, sulfate esters of alcohols synthesized with a Tiegler catalyst (e.g. ALFOL 1214; CONDEA), sulfate esters of alcohols synthesized by the oxo process (e.g. Dobanol 23, 25, 45, Mitsubishi Chemical; Tridecanol, Kyowa Hakko; Oxocol 1213, 1215, 1415, Nissan Chemical; Diadol 115-L, 115H, 135, Mitsubishi Chemical). A specific example of sulfate esters of said higher alkyl ethers includes lauryl alcohol-EO (2 moles) adduct sulfate ester and octyl alcohol-EO (3 moles) adduct sulfate ester, among others. A specific example of the sulfated oils includes sulfation products of castor oil, peanut oil, olive oil, rapeseed oil, beef tallow, wool fat, etc. A specific example of the sulfated fatty acid esters includes sulfation products of butyl oleate and butyl ricinolate, among others. A specific example of the sulfated olefins includes Teepol (Shell), among others.

The carboxymethyl compounds include carboxymethylated $C_{8-16}$ aliphatic alcohols (e.g. carboxymethylated octyl alcohol, carboxymethylated decyl alcohol, carboxymethylated lauryl alcohol, carboxymethylated Dobanol 23, carboxymethylated Tridecanol) and carboxymethylated $C_{8-16}$ aliphatic alcohol-EO (1 to 10 moles) adducts [e.g. carboxymethylated octyl alcohol-EO (3 moles) adduct, carboxymethylated lauryl alcohol-EO (4 moles) adduct, carboxymethylated Dobanol 23-EO (3 moles) adduct, carboxymethylated Tridecanol-EO (5 moles) adduct].

The phosphate esters include phosphate esters of higher alcohols ($C_{8-18}$) (e.g. lauryl alcohol phosphoric monoester, lauryl alcohol phosphoric diester, etc.) and phosphate esters of higher alcohols ($C_{8-18}$) or alkyl($C_{1-18}$)phenol-EO (1 to 10 moles) adducts [e.g. oleyl alcohol-EO (5 moles) adduct phosphoric monoester].

The inorganic acids such as hydrochloric acid, sulfuric acid, phosphoric acid, etc. are too irritating to the teats to be judiciously chosen.

The iodine carrier (D) includes a nonionic surfactant (D1) the HLB number of which may range from 6 to 15. In the above and following descriptions, HLB represents Griffin's HLB.

$$\text{(Griffin's HLB} = [\text{surfactant} \cdot \text{EO weight/surfactant molecular weight}] \times 20)$$

The preferred example of (D1) is one or more nonionic surfactants selected from the group consisting of polyethylene glycol fatty acid esters (D11) and nonionic surfactants (D12), (D13) and (D14) represented by the following general formulas (1), (2) and (3), respectively. From the standpoint of assuring an effective viscosity for sustained bactericidal effects even after dilution at a high dilution factor, more preferred is (D13) and/or (D14) or a combination of not less than 20 weight %, particularly not less than 50 weight %, of (D13) and/or (D14) and not more than 80 weight %, particularly not more than 50 weight %, of one or more other nonionic surfactants. Particularly preferred is (D14).

$$R^1O[(C_2H_4O)_n/(R^2O)_m]H \tag{1}$$

[wherein $R^1$ represents a straight-chain or branched-chain alkyl or alkenyl group of 8 to 22 carbon atoms or an aryl group having a straight-chain or branched chain alkyl or alkenyl group of 8 to 18 carbon atoms; $R^2$ represents a propylene group; n represents an integer of 2 to 30; m represents an integer of 0 to 30. When $m \neq 0$, $(C_2H_4O)_n/(R^2O)_m$ represents block addition or random addition]

$$R^3O-[(C_2H_4O)_p/(R^4O)_q]-(C_2H_4O)_r-H \tag{2}$$

[wherein $R^3$ represents an alkyl, alkenyl or cycloalkyl group of 8 to 24 carbon atoms; $R^4$ represents an alkylene group containing not less than 3 carbon atoms; p represents an integer of 0 to 4; q represents an integer of 1 to 3; r represents an integer of 1 to 80; (p+q+r) is an integer of 3 to 81 and (p+r)/(p+q+r) is not less than 0.5; when $p \neq 0$, $[(C_2H_4O)_p/(R^4O)_q]$ represents block addition or random addition]

$$R^5O-(C_2H_4O)_s-H \tag{3}$$

[wherein $R^5$ represents an alkyl, alkenyl or cycloalkyl group of 8 to 24 carbon atoms; s represents an integer of 3 to 80]

The fatty acids constituting said polyethylene glycol fatty acid esters (D11) include acids each having a straight-chain or branched-chain alkyl group of 8 to 24 carbon atoms (e.g. lauric acid, myristic acid, palmitic acid, stearic acid, isostearic acid) and acids each having a straight-chain or branched-chain alkenyl group of 8 to 24 carbon atoms (e.g. oleic acid), among others. The molecular weight of the polyethylene glycol is generally 100 to 2000, preferably 400 to 1000.

A specific example of (D11) includes the reaction products of said fatty acids with said polyethylene glycol. Among these, preferred are polyethylene glycol monostearate, polyethylene glycol distearate, polyethylene glycol monolaurate, and polyethylene glycol dilaurate. Particularly preferred is polyethylene glycol (Mw: 400 to 600) monostearate.

The nonionic surfactant (D12) of the above general formula (1) can be obtained by the addition reaction of 2 to 30 moles of EO and 0 to 30 moles of propylene oxide (hereinafter referred as to PO) to the monool $R^1OH$. Here, the addition of EO and PO may be block addition or random addition and, in the case of block addition, the first to be added may be whichever of EO and PO.

The monool residue $R^1$ is a straight-chain or branched-chain alkyl or alkenyl group of 8 to 22 carbon atoms or an aryl group having a straight-chain or branched-chain alkyl or alkenyl group of 8 to 18 carbon atoms; $R^2$ represents a propylene group; n represents an integer of usually 2 to 30, preferably 5 to 20; m represents an integer of usually 0 to 30, preferably 0 to 5; and when $m \neq 0$, $[(C_2H_4O)_n/(R^2O)_m]$ represents block addition or random addition.

A specific example of (D12) includes polyoxyalkylene($C_2$-$C_3$) alkyl($C_8$-$C_{22}$) ethers [e.g. polyoxyethylene lauryl ether, polyoxyethylene myristyl ether, polyoxyethylene stearyl ether, poly(oxyethylene-co-oxypropylene) lauryl ether] and polyoxyalkylene($C_2$-$C_3$) alkyl($C_8$-$C_{18}$) phenyl ethers (e.g. polyoxyethylene octylphenyl ether, polyoxyethylene nonylphenyl ether, poly(oxyethylene-co-oxypropylene) octylphenyl ether and poly(oxyethylene-co-oxypropylene) nonylphenyl ether), among others.

Among these, preferred are polyoxyethylene (7 to 11 moles added)nonylphenyl ether and polyoxyethylene (3 to 9 moles added) lauryl ether.

The nonionic surfactant (D13) of the above general formula (2) is one or a mixture of two or more aliphatic alcohol alkylene oxide adducts obtainable by the addition reaction of $C_{2-4}$ alkylene oxides to an aliphatic alcohol (a) (inclusive of alicyclic alcohols).

In the above general formula (2), $R^3$ represents an aliphatic alcohol residue and, from the standpoint of the viscosity of the diluted solution, is preferably an aliphatic or alicyclic hydrocarbon group containing generally 8 to 24 carbon atoms, preferably 12 to 18 carbon atoms. The aliphatic hydrocarbon group mentioned above includes straight-chain and/or branched-chain saturated or unsaturated aliphatic hydrocarbon groups (alkyl, alkenyl and alkadienyl); the alicyclic hydrocarbon group mentioned above includes cycloalkyl groups and polycyclic hydrocarbon groups. $R^3$ may be straight-chain, branched-chain or alicyclic, and may further be a mixture of two or more such groups.

To mention specific examples of $R^3$, the alkyl mentioned above includes octyl, nonyl, decyl, lauryl, tridecyl, myristyl, cetyl, stearyl, nonadecyl, 2-ethylhexyl and 2-ethyloctyl, among others. The alkenyl includes octenyl, decenyl, dodecenyl, tridecenyl, pentadecenyl, oleyl, gadoleyl and linoleyl, among others. The alkadienyl group includes linoleyl. The cycloalkyl includes ethylcyclohexyl, propylcyclohexyl, octylcyclohexyl and nonylcyclohexyl, among others. Said polycyclic hydrocarbon group includes adamantyl.

The aliphatic alcohol (a) for use in the present invention includes an alcohol giving said residue $R^3$ and having generally 8 to 24 (preferably 12 to 18) carbon atoms, and may be a natural alcohol or a synthetic alcohol (Ziegler process alcohol, oxo process alcohol, etc.).

A specific example thereof includes saturated aliphatic alcohols such as octyl alcohol, nonyl alcohol, decyl alcohol, undecyl alcohol, dodecyl alcohol, tridecyl alcohol, myristyl alcohol, cetyl alcohol, stearyl alcohol, nonadecyl alcohol, etc.; unsaturated aliphatic alcohols such as octenyl alcohol, decenyl alcohol, dodecenyl alcohol, tridecenyl alcohol, pentadecenyl alcohol, oleyl alcohol, gadoleyl alcohol, linoleyl alcohol, etc.; and alicyclic alcohols such as ethylcyclohexyl alcohol, propylcyclohexyl alcohol, octylcyclohexyl alcohol, nonylcyclohexyl alcohol, adamantyl alcohol, etc. These aliphatic alcohols are preferably primary or secondary, and the primary alcohols are particularly preferred. The alkyl moiety may be straight-chain or branched-chain.

In the above general formula (2), the ($C_2H_4O$) moiety is formed by the addition of EO. $R^4$ represents an alkylene group containing not less than 3 carbon atoms, preferably 3 to 8 carbon atoms, more preferably 3 carbon atoms, and the ($R^4O$) moiety is formed by the addition of an alkylene oxide containing 3 or more carbon atoms. The alkylene oxide mentioned just above includes PO, 1,2- or 2,3-butylene oxide, tetrahydrofuran, styrene oxide, etc. Preferred is PO.

In the above general formula (2), p represents an integer of generally 0 to 4, preferably 0 to 3, more preferably 1 to 3; q represents an integer of generally 1 to 3, preferably 1 or 2; r represents an integer of generally 1 to 80 and, from the view point of the viscosity of the diluted solution, is preferably 2 to 70, more preferably 3 to 40; (p+q+r) is generally an integer of 3 to 81, preferably an integer of 3 to 71 from the standpoint of diluted viscosity, (p+r)/(p+q+r) is generally not less than 0.5, preferably 0.7 to 0.99. The $[(C_2H_4O)_p/(C_3H_6O)_q]$ moiety may represent block addition, random addition, or mixed addition. Preferred is block addition.

The weight average molecular weight (Mw) of (D13) is generally 170 to 5,000, preferably 250 to 1,200, from the standpoint of the viscosity of the diluted solution (hereinafter referred to as "diluted viscosity"). For the determination of molecular weight distribution, gel permeation chromatography (GPC) is used. This applies hereinbelow. From the standpoint of diluted viscosity, it is preferable that the ratio of weight average molecular weight (Mw) to number average molecular weight (Mn), i.e. Mw/Mn, satisfy the following formula (4) or (5).

$$Mw/Mn \leq 0.030 \times Ln(v) + 1.010 \text{ (where } v < 10) \tag{4}$$

$$Mw/Mn \leq 0.026 \times Ln(v) + 1.139 \text{ (where } v \geq 10) \tag{5}$$

In the above formulas, $Ln(v)$ represents a natural logarithm of $v$, where $v$ represents the average number of moles of alkylene oxide added to each mole of aliphatic alcohol (a), and corresponds to the average sum of p, q and r, which represent the numbers of moles of the respective alkylene oxides added, in general formula (2).

When the above formula (4) or (5) is satisfied, the molecular weight distribution is sufficiently narrow so that an effective level of viscosity is assured even at a high dilution factor with greater certainty for sustained bactericidal activity. More preferably, Mw/Mn satisfies the following formula (4') or (5'). $Mw/Mn \leq 0.031 \times Ln(v) + 1.000$ (where $v < 10$) (4') $Mw/Mn \leq -0.026 \times Ln(v) + 1.129$ (where $v \geq 10$) (5')

Referring, further, to (D13), the distribution constant c can be calculated from the following equation (6) based on Weibull distribution law, and from the standpoint of diluted viscosity, the value of c is preferably not more than 1.0, more preferably not more than 0.9, particularly preferably not more than 0.7. Referring to the equation (6), the smaller the distribution constant c is, that is to say the lower the unreacted aliphatic alcohol content is, the narrower is the molecular weight distribution. This equation holds when the amount of unreacted aliphatic alcohol (a) is not less than its detection limit (0.001 weight%) and, in the case of (D13), is applicable when the average number of moles of alkylene oxide added is up to about 10 moles.

When the value of c is 1.0 or less, the molecular weight distribution is narrow so that an effective level of viscosity for expressing a sufficient bactericidal effect can be assured with greater certainty even at a high dilution factor. $c = (v + n_0/n_{00} - 1)/[Ln(n_{00}/n_0) + n_0/n_{00} - 1]$ (6)

In the above equation (6), $Ln(n_{00}/n_0)$ represents a natural logarithm of $(n_{00}/n_0)$, where $n_{00}$ represents the number of moles of the aliphatic alcohol (a) and $n_o$ represents the number of moles of the unreacted aliphatic alcohol (a).

The nonionic surfactant (D14) of the above general formula (3) is a species or a mixture of 2 or more species of the aliphatic alcohol-EO adduct produced by the addition reaction of EO to said aliphatic alcohol (a) (inclusive of alicyclic alcohol).

In the above general formula (3), $R^5$ represents an aliphatic alcohol (a) residue and, from the standpoint of diluted viscosity, is preferably selected from among aliphatic or alicyclic hydrocarbon groups containing generally 8 to 24 carbon atoms, preferably 12 to 18 carbon atoms. The aliphatic hydrocarbon groups mentioned just above include straight-chain and/or branched-chain saturated or unsaturated aliphatic hydrocarbon groups (alkyl, alkenyl, alkadienyl). The alicyclic hydrocarbon groups include cycloalkyl groups and polycyclic hydrocarbon groups. $R^5$ may be straight-chain, branched-chain or cyclic, or may represent a mixture of 2 or more such groups.

$R^5$ includes the same specific groups as mentioned above for $R^3$.

From the standpoint of diluted viscosity, s in the above general formula (3) is an integer of generally 3 to 80, preferably 3 to 40.

The weight average molecular weight (Mw) of (D14), also from the standpoint of diluted viscosity, is generally 170 to 5,000, preferably 250 to 1,200. It is preferred that the ratio of weight average molecular weight (Mw) to number average molecular weight (Mn), i.e. Mw/Mn, satisfies the following formula (7) or (8) from the standpoint of diluted viscosity.

$$Mw/Mn \leq 0.020 \times Ln(v) + 1.010 \text{ (where } v < 10) \tag{7}$$

$$Mw/Mn \leq 0.026 \times Ln(v) + 1.116 \text{ (where } v \geq 10) \tag{8}$$

In the above formulas, $v$ represents the average number of moles of alkylene oxide added per mole of aliphatic alcohol (a), and corresponds to the average value of s representing the number of moles of EO added in the above general formula (3).

When the above formula (7) or (8) is satisfied, that is to say the molecular weight distribution is narrow, an effective level of viscosity for assuring a sufficient bactericidal effect can be maintained with greater certainty even at a high dilution factor.

More preferably, Mw/Mn satisfies the following formula (7') or (8').

$$Mw/Mn \leq 0.018 \times Ln(v) + 1.015 \text{ (where } v < 10) \tag{7'}$$

$$Mw/Mn \leq -0.023 \times Ln(v) + 1.113 \text{ (where } v \geq 10) \tag{8'}$$

Moreover, when the distribution constant c can be determined from the following formula (6) based on Weibull distribution law, the value of c for (D14) is preferably not more than 1.0, more preferably not more than 0.9, particularly not more than 0.7, from the standpoint of diluted viscosity. When, referring to the formula (6), the distribution constant c is small, that is to say the unreacted aliphatic alcohol content is low, the narrower is the molecular weight distribution.

This formula holds when the amount of unreacted aliphatic alcohol (a) is not less than the detection limit (0.001 weight %) and, in the case of (D14), the formula is applicable when the average number of moles of EO is up to about 10 moles.

When the value of c is not more than 1.0, that is to say the molecular weight distribution is narrow, an effective viscosity level for sustained bactericidal activity can be assured even at a high dilution factor.

$$c = (v + n_0/n_{00} - 1)/[Ln(n_{00}/n_0) + n_0/n_{00} - 1] \tag{6}$$

In the above formula (6), $Ln(n_{00}/n_0)$ represents a natural logarithm of $(n_{00}/n_0)$.

The method of producing the polyethylene glycol fatty acid ester (D11) for use in the present invention is not particularly restricted but includes the following processes.

[1] Using potassium hydroxide as the catalyst, EO is added to ethylene glycol. Then, an esterification reaction with a higher fatty acid is carried out.

[2] Using potassium hydroxide as the catalyst, EO is added to a higher fatty acid.

Of the above processes, the process [1] is preferred.

The method of producing the nonionic surfactant (D12) of the general formula (1) is not particularly restricted but includes the following processes, among others.

[1] Using potassium hydroxide as the catalyst, PO is added to an aliphatic alcohol or an alkylaryl, followed by block addition of EO.

[2] Using potassium hydroxide as the catalyst, EO is added to an aliphatic alcohol or an alkylaryl, followed by block addition of PO.

[3] Using potassium hydroxide as the catalyst, a mixture of EO and PO is reacted with an aliphatic alcohol or an alkylaryl for random addition.

Of the above processes, the processes [1] and [2] are preferred and the process [1] is particularly preferred.

The nonionic surfactants (D13) and (D14) represented by the general formulas (2) and (3), respectively, for use in the present invention can be produced by the following technology. Thus, an aliphatic alcohol alkylene oxide adduct is first prepared by adding an average of 1 to 2.5 moles of an alkylene oxide (b1) with an aliphatic alcohol (a) in the presence of a catalyst (d) capable of giving an adduct having a distribution constant value of c≤1.0 as calculated from the equation (6) based on the Weibull distribution law. This adduct (e) is further subjected to addition reaction with an alkylene oxide (b2) containing 2 or more carbon atoms in the presence of an alkaline catalyst (f), whereby an aliphatic alcohol alkylene oxide adduct with a narrow molecular weight distribution can be obtained.

$$c = (v + n_0/n_{00} - 1)/[Ln(n_{00}/n_0) + n_0/n_{00} - 1] \tag{6}$$

[wherein v represents the average number of moles of alkylene oxide added per mole of aliphatic alcohol (a); $n_{00}$ represents the number of moles of the aliphatic alcohol (a) and $n_0$ represents the number of moles of the unreacted aliphatic alcohol (a)].

The aliphatic alcohol (a) includes the same alcohols as mentioned for (D13) and (D14).

The alkylene oxide mentioned for (b1) and (b2) includes alkylene oxides containing 2 or more carbon atoms, preferably 2 to 8 carbon atoms. To mention specific examples, EO, PO, 1,2-or 2,3-butylene oxide, tetrahydrofuran, styrene oxide, etc. can be used and 2 or more of them may be used in combination. When 2 or more different species are used, the mode of addition may be block or random. Among the alkylene oxides mentioned above, preferred are EO and PO.

As the catalyst (d), use is made of a catalyst such that the distribution constant c of the resulting alkylene oxide adduct will be 1.0 or less, preferably not more than 0.7, more preferably not more than 0.45.

Said catalyst assuring a molecular weight distribution constant of c≤1.0 includes, for example, perhalic acids (inclusive of salts thereof), sulfuric acid (inclusive of its salts) and nitric acid (inclusive of its salts). The metals with which said acids form salts are not particularly restricted but are preferably metals other than alkali metals, which are divalent or trivalent. Preferred metals are Mg, Ca, Sr, Ba, Zn, Co, Ni, Cu and Al. More preferred metals are Mg, Zn, Ca, Sr, Ba and Al. Particularly preferred are Mg, Zn and Al. The halogens of said perhalic acids (salts) include chlorine, bromine and iodine. Preferred is chlorine. Therefore, for (d), perchlorates of divalent or trivalent metals are preferred and the perchlorate of a metal selected from among Mg, Zn and Al is still more preferred. Moreover, for (d), a divalent or trivalent metal alcoholate may be additionally used in combination. The alkyl moiety of said metal alcoholate includes lower ($C_{1-4}$)alkyl groups which can be easily distilled off in the form of the corresponding alcohols and the same alkyl groups as the alkyl groups constituting the starting material aliphatic alcohols used. These catalysts may be used singly but it is preferable to use 2 or more kinds of catalysts in combination.

The amount of use of the catalyst (d) is preferably 0.001 to 1 weight part per 100 weight parts of the sum of (a) and (b1) from the standpoint of reaction rate and from economic considerations. More preferred amount is 0.003 to 0.8 weight part. Particularly preferred amount is 0.005 to 0.5 weight part.

The catalyst to be used in the addition of alkylene oxide (b2) to the alkylene oxide adduct (e) formed by said addition reaction of (a) with (b1) is an alkaline catalyst (f). The alkaline catalyst (f) includes hydroxides of alkali metals and those of alkaline earth metals, such as lithium hydroxide, sodium hydroxide, potassium hydroxide, cesium hydroxide, magnesium hydroxide, calcium hydroxide, barium hydroxide, etc., however potassium hydroxide and cesium hydroxide are particularly preferred.

From the standpoint of reaction rate and from economic considerations, preferred amount of use of the catalyst (f) is 0.0001 to 1 weight part per 100 weight parts of (e) and (b2) combined. More preferred amount is 0.001 to 0.8 weight part.

The conditions of reacting (a) with (b1) may for example comprise blending (a) and (b), performing nitrogen purging, feeding a necessary amount of (b1) under -0.8 to 5 kgf/cm$^2$G at 80 to 200 °C, and carrying out an aging reaction at 80 to 200 °C until the pressure within the reaction system has steadied.

To the alkylene oxide adduct (e) thus obtained is added the alkaline catalyst (f), and the alkylene oxide (b2) is further reacted in the same manner as above to give the objective aliphatic alcohol alkylene oxide adduct.

The proportion of iodine (A) based on the weight of the composition is generally 0.05 to 10%, preferably 0.1 to 5%, from the standpoint of bactericidal efficacy, irritation to the teats, and production cost.

The proportion of the iodide (B) based on the weight of the composition is generally 0.05 to 5%, preferably 0.2 to 3%, from the standpoint of the stability of iodine and cost.

The proportion of the organic acid (C) based on the weight of the composition is generally 0.05 to 15%, preferably 0.1 to 10%, from the standpoint of the stability of the composition, bactericidal efficacy, and irritation to the teats.

The proportion of the iodine carrier (D) based on the weight of the composition is generally 20 to 90%, preferably 30 to 70%, from the standpoint of effective iodine deposition and the rate of dissolution in water.

The proportion of water based on the weight of the composition is generally 5 to 50%, preferably 10 to 40%, from the standpoint of the solubilities of the respective compositions and the viscosity of the final dilution.

The weight ratio (A)/(D) of iodine (A) and iodine carrier (D) is generally 20/80 to 1/99, preferably 10/90 to 2/98, from the standpoint of cost and bactericidal efficacy.

The viscosity of a 10-fold dilution of the composition of the present invention is generally 20 to 3000 mm$^2$/s, preferably 50 to 500 mm$^2$/s. If the viscosity of such a 10-fold dilution is less than 20 mm$^2$/s, the effective amount of iodine which can be deposited on the teats will be small as compared with the amount of iodine deposited from a 4-fold dilution of the conventional product. If the viscosity exceeds 3000 mm$^2$/s, more than the necessary amount of iodine will be deposited on the teats so that the teats will be excessively irritated.

The (V2)/(V1) ratio of the viscosity (V2) of the stock solution to the viscosity (V1) of a 10-fold dilution of the composition is generally 0.05 to 20, preferably 1.0 to 10. In order to bring down the (V2)/(V1) ratio to less than 0.05, it will be necessary to add an alcohol or other organic solvent to the composition but, then, the risk for ignition will be inevitable. On the other hand, if the (V2)/(V1) ratio exceeds 20, the composition itself will become too viscous to be smoothly taken out from a container and, in addition, it takes a long time for the composition to be dissolved in water for dilution, thus detracting from workability.

In order to increase the viscosity of the dilution, it might be contemplated to add a macromolecular thickener such as polyvinyl alcohol, xanthan gum or the like. However, since such addition of a thickener increases the viscosity of the stock solution to cause the problems mentioned above as well as the disadvantage of solidification in the winter months in frigid areas.

Where necessary, the iodophor composition of the present invention may be supplemented with a humectant in a quantity not affecting the efficacy of the invention. Said humectant includes glycerin, polyethylene glycol, sorbitol, propylene glycol and 1,3-butylene glycol, among others. Among these, preferred are polyethylene glycol and propylene glycol.

The method of producing the composition of the invention is not particularly restricted but, for example, the composition can be prepared by adding and dissolving said iodine (A), iodide (B) and organic acid (C) in an aqueous solution of said iodine carrier (D) at room temperature. It may also be a sound practice to heat the mixture at 50 to 60 °C to expedite dissolution, where necessary.

The representative mode of use of the composition of the present invention comprises diluting the composition so that the effective amount of iodine will be generally 1000 to 5000 ppm, preferably 1500 to 3000 ppm, and dipping teats and the udder in the diluted composition for a brief time (for example, 1 to 5 seconds).

The composition of the present invention finds application to farm animals and is very useful for the topical antisepsis of the animal tissues, particularly antisepsis of the udder and teats of farm animals.

BEST MODE FOR CARRYING OUT THE INVENTION

The following example is further illustrative of the present invention but by no means defining the scope of the invention. In the example, the formulating amounts in parts are all by weight.

According to the formulations shown in Table 1, the iodophore compositions for prevention of mastitis of Examples 1 to 5 and Comparative Examples 1 and 2 were prepared. The compositions indicated by abbreviations in Table 1 are as follows.

| | |
|---|---|
| A: | iodine |
| B: | sodium iodide |
| C: | citric acid |
| D111: | polyethylene glycol(Mw 400) stearate (HLB 11.9) |
| D121: | polyoxyethylene nonylphenyl ether (EO 8.5 mole adduct, HLB 12.6) |
| D122: | polyoxyethylene alkyl ether (EO 5.0 mole adduct, HLB 10.5) |
| D131: | poly(oxyethylene-co-oxypropylene) alkyl ether (EO 9.0 mole/PO 1.0 mole adduct, HLB 11.8, Mw/Mn 1.067, unreacted alcohol 0.003%) |
| D141: | polyoxyethylene alkyl ether (EO 7.0 mole adduct, HLB 11.7, Mw/Mn 1.045, unreacted alcohol 0.02%) |
| E1: | polyethylene glycol (Mw 400) |
| E2: | propylene glycol |
| F1: | poly(oxyethylene-co-oxypropylene) block polymer (EO 25 mole/PO 30 mole adduct, HLB 7.7) |

Table 1

| Composition | Example | | | | | | | Compar. Ex. | |
|---|---|---|---|---|---|---|---|---|---|
| | 1 | 2 | 3 | 4 | 5 | 6 | 7 | 1 | 2 |
| A | 3 | 3 | 3 | 3 | 3 | 3 | 3 | 3 | 3 |
| B | 1.5 | 1.5 | 1.5 | 1.5 | 1.5 | 1.5 | 1.5 | 1.5 | 1.5 |
| C | 5 | 5 | 5 | 5 | 5 | 5 | 5 | 5 | 5 |
| D111 | 50 | | | | | | | | |
| D121 | | 50 | 50 | 30 | | | | 15 | |
| D122 | | | | | 50 | | | | |
| D131 | | | | | | 50 | | | |
| D141 | | | | | | | 30 | | |
| E1 | 12.5 | 12.5 | | 12.5 | 12.5 | 12.5 | 12.5 | 12.5 | 12.5 |
| E2 | | | 12.5 | | | | | | |
| F1 | | | | | | | | | 50 |
| Water | 28 | 28 | 28 | 48 | 28 | 28 | 48 | 63 | 28 |
| Total | 100 | 100 | 100 | 100 | 100 | 100 | 100 | 100 | 100 |

The antimastitis iodophore compositions prepared in Examples 1 to 7 and Comparative Examples 1 and 2 were subjected to the following performance evaluations. Viscosity measurements were performed for each stock solution and a 10-fold dilution of the stock solution in tap water, and the effective amount of iodine deposited was determined for the 10-fold dilution. The Ubbelohde viscometer was used for the above viscosity measurements (measuring temperature: 20 °C; unit: mm$^2$/s).

[Evaluation of effective Iodine deposition amount]

A 50 ml glass common-stoppered centrifuge tube (hereinafter referred to briefly as centrifuge tube) set securely on a test tube stand was immersed up to the graduation "10" in the 10-fold dilution for 5 seconds. The tube was taken out and allowed to stand for 1 minutes.

Then, the centrifuge tube was washed thoroughly with deionized water and the effective iodine amount in the washes was determined with an automatic titrimeter (titrant: 0.01 N sodium thiosulfate).

Table 2

| | Example | | | | | | | Compar. Ex. | |
|---|---|---|---|---|---|---|---|---|---|
| | 1 | 2 | 3 | 4 | 5 | 6 | 7 | 1 | 2 |
| Viscosity of dilution (V1) | 257 | 159 | 140 | 25 | 40 | 2150 | 61 | 13 | 2 |
| Viscosity of stock solution (V2) | 479 | 420 | 318 | 240 | 116 | 134 | 200 | 150 | 1273 |
| (V2)/(V1) | 1.86 | 2.64 | 2.27 | 9.60 | 2.90 | 0.06 | 3.28 | 11.5 | 637 |
| Effective Iodine deposition amount | 1.38 | 0.95 | 0.82 | 0.20 | 0.24 | 3.05 | 0.80 | 0.10 | 0.01 |

It will be apparent from Table 2 that the antimastitis iodophor composition of the present invention retains a satisfactory viscosity and is capable of providing an effective amount of iodine even after 10-fold dilution.

Then, a 10-fold dilution of the composition of the invention was compared with a 4-fold dilution of the conventional antimastitis iodophor composition for bactericidal efficacy. The result indicated that the two solutions were equipotent in bactericidal activity.

Thus, using the above composition according to Example 1 as the composition of the invention and "Coatlac" (manufactured by Sanyo Chemical Industries and distributed by Fujisawa Pharmaceutical Co.) as the conventional antimastitis iodophor composition together with sterile water, a comparative teat dip test was performed.

The following test protocol was used.

1. Preparation of the teat

The teat isolated from a slaughtered cow was hung on a cane pole and disinfected with 70% ethanol for 10 seconds, rinsed with sterile water, and dried before commencement of the test.

2. Dipping in a bacterial suspension

Substantially the whole of the teat was immersed in a *Staphylococcus aureus* cell suspension for a few seconds and, then, allowed to sit for about 10 minutes.

3. Inspection before dipping

Using an absorbent cotton applicator with its tip wetted with sterile saline, the left lateral surface of the teat treated with the bacterial suspension was wiped, and the cotton applicator was placed in a test tube containing 10 ml of saline.

4. Dipping in the drug solution

Using the 10-fold dilution of Example 1, a 4-fold dilution of Coatlac or sterile water, the teat was submerged for a few seconds in the test solution, taken out and allowed to sit for about 5 minutes.

5. Inspection after dipping

The right lateral surface of each teat was wiped in the same manner as in the inspection before dipping to prepare test samples.

6. Determination of the number of bacteria

The samples prepared before and after dipping were respectively smeared on agar plates and incubated at 37 °C for 24 hours. Then, the number of colonies grown on each agar plate was determined.

The bacterial count prior to dipping was 1000 cfu/ml but no bacteria were detected from the teats dipped in the 10-fold dilution of Example 1 or the 4-fold dilution of Coatlac. From the teat dipped in sterile water, about 300 cfu/ml of bacteria were detected. It was, therefore, clear that, in bactericidal activity, the composition of the invention is at least equivalent to the conventional product.

INDUSTRIAL APPLICABILITY

The composition of the present invention has the following performance characteristics.

1. The composition has good workability with a viscosity comparable to that of the conventional product and yet exhibits a viscosity even after high-factor dilution which is at least equal to the conventional dilution so that an effective amount of iodine can be deposited on the teats of animals. This high dilution factor feature contributes to cost reduction.
2. In the topical antisepsis of farm animals, particularly in the prevention of mastitis in farm animals, an improved antibacterial effect can be assured even after dilution at a high dilution factor.
3. The composition is hard to freeze even in cold climates and, in addition, superior in workability because it can be supplied in containers of reduced size.

Having the above performance characteristics, the composition of the present invention is very useful for the topical antisepsis of farm animals, especially as an iodophor composition for the prevention of mastitis in cows and other animals.

**Claims**

1.  An iodophore composition for prevention of mastitis comprising iodine(s) (A), an iodide(s) (B), an organic acid(s) (C), an iodine carrier(s) (D) and water,
    wherein the viscosity (V1) of a 10-fold dilution of the composition is 20 to 3000 $mm^2$/s and

    the ratio of the viscosity (V2) of the composition as such to the viscosity (V1) of the dilution thereof, i.e. (V2)/(V1), is 0.05 to 20.

2.  The composition according to Claim 1
    wherein the iodine carrier (D) is a nonionic surfactant (D1) having an HLB number of 6 to 15.

3.  The composition according to Claim 2
    wherein the nonionic surfactant (D1) is at least one member selected from the group consisting of a polyethylene glycol fatty acid ester (D11) and nonionic surfactants (D12), (D13) and (D14) represented by the following general formulas (1), (2) and (3), respectively:

$$R^1O[(C_2H_4O)_n/(R^2O)_m]H \qquad (1)$$

wherein $R^1$ represents a straight-chain or branched-chain alkyl or alkenyl group of 8 to 22 carbon atoms or an aryl group having a straight-chain or branched chain alkyl or alkenyl group of 8 to 18 carbon atoms; $R^2$ represents a propylene group; n represents an integer of 2 to 30; m represents an integer of 0 to 30; when $m \neq 0$, $(C_2H_4O)_n/(R^2O)_m$ represents block addition or random addition;

$$R^3O\text{-}[(C_2H_4O)_p/(R^4O)_q]\text{-}(C_2H_4O)_r\text{-}H \qquad (2)$$

wherein $R^3$ represents an alkyl, alkenyl or cycloalkyl group of 8 to 24 carbon atoms; $R^4$ represents an alkylene

group containing not less than 3 carbon atoms; p represents an integer of 0 to 4; q represents an integer of 1 to 3; r represents an integer of 1 to 80; (p+q+r) is an integer of 3 to 81 and (p+r)/(p+q+r) is not less than 0.5; when p ≠ 0, $[(C_2H_4O)_p/(R^4O)_q]$ represents block addition or random addition;

$$R^5O\text{-}(C_2H_4O)_s\text{-}H \qquad (3)$$

wherein $R^5$ represents an alkyl, alkenyl or cycloalkyl group of 8 to 24 carbon atoms; s represents an integer of 3 to 80.

4. The composition according to Claim 3
wherein the (D13) is a nonionic surfactant such that the ratio of weight average molecular weight (Mw) to number average molecular weight (Mn), i.e. Mw/Mn, satisfies the following formula (4) or (5) and the distribution constant c as calculated from the following equation (6) based on the Weibull distribution law is not more than 1.0:

$$Mw/Mn \leq 0.030 \times Ln(v) + 1.010 \text{ (where } v < 10) \qquad (4)$$

$$Mw/Mn \leq -0.026 \times Ln(v) + 1.139 \text{ (where } v \geq 10) \qquad (5)$$

wherein v represents the average number of moles of alkylene oxide added to each mole of aliphatic alcohol (a), and corresponds to the average value of (p+q+r) in the above general formula (2);

$$c = (v + n_0/n_{00} - 1)/[Ln(n_{00}/n_0) + n_0/n_{00} - 1] \qquad (6)$$

wherein v represents the average number of moles of alkylene oxide added to each mole of aliphatic alcohol (a), and corresponds to the average value of (p+q+r) in the above general formula (2), $n_{00}$ represents the number of moles of the aliphatic alcohol (a) and $n_0$ represents the number of moles of the unreacted aliphatic alcohol (a).

5. The composition according to Claim 3
wherein the (D14) has the ratio of weight average molecular weight (Mw) to number average molecular weight (Mn), i.e. Mw/Mn, satisfying the following formula (7) or (8) and not more than 1.0 of the distribution constant c as calculated from the following equation (6) based on the Weibull distribution law:

$$Mw/Mn \leq 0.020 \times Ln(v) + 1.010 \text{ (where } v < 10) \qquad (7)$$

$$Mw/Mn \leq -0.026 \times Ln(v) + 1.116 \text{ (where } v \geq 10) \qquad (8)$$

wherein v represents the average number of moles of alkylene oxide added to each mole of aliphatic alcohol (a), and corresponds to s in the above general formula (3);

$$c = (v + n_0/n_{00} - 1)/[Ln(n_{00}/n_0) + n_0/n_{00} - 1] \qquad (6)$$

wherein v represents the average number of moles of alkylene oxide added to each mole of aliphatic alcohol (a), $n_{00}$ represents the number of moles of the aliphatic alcohol (a) and $n_0$ represents the number of moles of the unreacted aliphatic alcohol (a).

6. The composition according to any of Claims 1 to 5
wherein, based on the weight of the total composition, (A) accounts for 0.05 to 10%, (B) accounts for 0.05 to 5%, (C) accounts for 0.05 to 15%, (D) accounts for 20 to 90%, and water accounts for 5 to 50%.

7. The composition according to any of Claims 1 to 6
wherein the (A)/(D) ratio is 20/80 to 1/99 by weight.

## INTERNATIONAL SEARCH REPORT

| International application No. |
| --- |
| PCT/JP99/02328 |

**A. CLASSIFICATION OF SUBJECT MATTER**
Int.Cl$^6$ A61K33/18, A61K47/30

According to International Patent Classification (IPC) or to both national classification and IPC

**B. FIELDS SEARCHED**

Minimum documentation searched (classification system followed by classification symbols)
Int.Cl$^6$ A61K33/18, A61K47/30

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP, 3-77828, A (Tanabe Seiyaku Co., Ltd.), 3 April, 1991 (03. 04. 91), Full text (Family: none) | 1-7 |
| X | JP, 8-500364, A (West Agro Inc.), 16 January, 1996 (16. 01. 96), Full text & WO, 94/06444, A1 & US, 5368868, A & EP, 662836, A1 | 1-7 |

☐ Further documents are listed in the continuation of Box C.   ☐ See patent family annex.

| * Special categories of cited documents: | | "T" later document published after the international filing date or priority |
| --- | --- | --- |
| "A" document defining the general state of the art which is not considered to be of particular relevance | | date and not in conflict with the application but cited to understand the principle or theory underlying the invention |
| "E" earlier document but published on or after the international filing date | | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | | |
| "O" document referring to an oral disclosure, use, exhibition or other means | | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | | "&" document member of the same patent family |

| Date of the actual completion of the international search | Date of mailing of the international search report |
| --- | --- |
| 22 July, 1999 (22. 07. 99) | 3 August, 1999 (03. 08. 99) |

| Name and mailing address of the ISA/ Japanese Patent Office | Authorized officer |
| --- | --- |
| Facsimile No. | Telephone No. |

Form PCT/ISA/210 (second sheet) (July 1992)